# EUROPEAN PATENT APPLICATION

(11) **EP 1 419 749 A1**
(43) Date of publication of application: **19.05.2004**
(21) Application number: 03026084.8
(22) Date of filing: 12.11.2003
(51) Int. Cl.: A61F 2/30

(54) **Distal tip for the positioning of artificial joint stem**

(30) Priority: 18.11.2002 JP 2002333193
(71) Applicant: Katsuya, Toshinobu, Kobe-shi, Hyogo 658-0073 (JP)
(72) Inventor: Katsuya, Toshinobu, Kobe-shi, Hyogo 658-0073 (JP)
(74) Representative: Reinhard - Skuhra - Weise & Partner GbR

(57) **Abstract**

The present invention provides a distal tip for the positioning of artificial joint stem that is capable of reliably guiding the insertion of the stem of the cementless artificial joint into a bone canal and positioning the distal end portion of the stem that is inserted into the bone canal at a planned position, thereby to maintain the entire stem in a favorable posture in the bone canal, and prevent osteolysis and loosening of the artificial join even when the distal tip is used.

The distal tip 10 for the positioning of artificial joint stem is mounted near the distal end portion 2a of the stem of the cementless artificial joint thereby to guide the distal end portion 2a of the stem so as not to make direct contact with the inner surface of the bone canal 4 of the bone 3 when the stem 2 of the artificial joint is inserted into the bone canal 4, and make stable positioning of the stem distal end portion 2a when the insertion of the stem 2 is completed, wherein the distal tip 10 is made of a biodegradable and absorbable material.

## Description

### BACKGROUND OF THE INVENTION

### 1. Field of the Invention

The present invention relates to a distal tip for the positioning of artificial joint stem and, more particularly, to a distal tip for the positioning of a stem used in a cementless artificial joint.

### 2. Description of Related Art

Artificial joints can be roughly divided into a cemented artificial joint which is inserted in a bone canal that is filled with cement so that clearance between the bone and the stem of the artificial joint inserted therein is filled and fixed with the cement, and an artificial joint of cementless type that is designed for directly joining the stem of the artificial joint with the bone.

In the case of artificial joint of cementless type, stable connection of the artificial joint and the bone is achieved by joining with cancellous bone in the proximate portion of the stem, although the cancellous bone takes several months to grow at the earliest.

When an artificial joint of cementless type is applied to the hip joint, for example, a stem which is made thicker not only in the proximate portion but also in the distal portion has been used for femoral bone, in order to increase the occupancy ratio of the artificial joint stem in the bone canal of the femoral bone stem. Since the bone canal has anatomical curve, however, distal end of the artificial hip joint stem comes into contact with the bone inside the bone canal to cause concentration of stress at the contact point, resulting in osteolysis which leads to loosening of the artificial joint, pain and/or bone fracture.

In order to overcome the problem described above, an artificial joint having a stem that tapers off on the distal side has been provided. However, the stem which becomes thinner toward the distal end makes it difficult to precisely locate the narrower distal end portion of the stem at the center of the bone canal when inserting the stem into the bone canal, and the stem angle tends to take a varus position, valgus position, anteversion position or retroversion position in the bone canal. This situation also leads to osteolysis and loosening of the artificial joint.

In order to solve this problem, such a method has been provided as a metallic distal tip called the centralizer is mounted on the stem near the distal end thereof in a cementless artificial joint, and thereby the distal end of the stem is located at the center of bone canal. With this method, however, the distal tip mounted on the stem comes into contact with the bone to cause concentration of stress at the contact point, again resulting in osteolysis and loosening of the artificial joint.

With such a background as described above, it requires expedient to implant an artificial hip joint of cementless type, and tapered stems and straight stems without distal tips are used in implanting surgical operations at present.

Artificial joints of cemented type, on the other hand, is used in such a way as the bone canal is filled with cement so as to fix the stem inserted therein and the bone.

For the artificial joints of cemented type, too, such a stem is provided that has a distal tip, made of a plastic or cement of the same kind as the filler cement, is mounted as a centralzer near the distal end of the stem. Major role of the distal tip in the artificial joints of cemented type is to position the distal end of the stem at the center of the bone canal, thereby to evenly distribute the filler cement around it. In the artificial joints of cemented type, the inserted stem and the cement are integrated into a single block.

Japanese Unexamined Patent Publication (Kokai) No. 10-309297 discloses an invention related to an artificial joint of cemented type, wherein a plug made of a material which can be decomposed and absorbed in living tissue is inserted in the bone canal in advance, so that cement does not enter too deep in the bone canal when the bone canal is filled with the cement. However, the plug made of a biodegradable and absorbable material is an exclusive attachment for the artificial joints of cemented type, and is used for the purpose of preventing the cement from falling off.

With the artificial joint of cementless type, as described above, although it is made easier to position the distal end portion of the stem at the center of the bone canal by mounting the distal tip on the stem of the artificial joint, the distal tip makes contact with the bone over a long period of time so as to cause concentration of stress, thus resulting in osteolysis and loosening of the artificial joint.

In the case of the artificial joint of cemented type, on the other hand, since the stem is integrated with the filler cement, concentrated stress due to the use of the distal tip and the trouble such as osteolysis and loosening of the artificial joint do not pose significant problems from the first.

### BRIEF SUMMARY OF THE INVENTION

Accordingly, an object of the present invention is to eliminate the drawbacks and problems of the conventional cementless artificial joint described above and provide a distal tip for positioning of artificial joint stem that is capable of reliably guiding the insertion of the stem of the cementless artificial joint and positioning the distal end portion of stem that is inserted into the bone canal at a planned position, thereby to maintain the entire stem in a predetermined posture in the bone canal, and prevent osteolysis and loosening of the artificial join that have been taking place in the prior art from occurring even when the distal tip is used.

### BRIEF DESCRIPTION OF THE DRAWINGS

Fig. 1 is a sectional view showing the artificial joint stem having the distal tip of the present invention mounted thereon inserted in the bone canal of femoral bone.
Fig. 2 (A) through (F) are perspective views showing various embodiments of the distal tip employing fitting holes.
Fig. 3 (A) and (B) are perspective views showing embodiments of the distal tip of screw type.
Fig. 4 is a plan view showing a distal tip of an embodiment wherein fitting hole of the distal tip is offset from the center axis thereof.
Fig. 5 (A) and (B) are plan view and longitudinal sectional view, respectively, showing a distal tip of another embodiment wherein the fitting hole of the distal tip is offset from the center axis thereof.
Fig. 6 (A) and (B) are plan view and longitudinal sectional view, respectively, showing a distal tip of further another embodiment wherein the fitting hole of the distal tip is offset from the center axis thereof.
Fig. 7 (A) and (B) are plan view and longitudinal sectional view, respectively, showing a distal tip of further another embodiment wherein the fitting hole of the distal tip is offset from the center axis thereof.
Fig. 8 is a plan view showing an embodiment wherein a ridge protruding toward the center is formed on the inner circumferential surface of the fitting hole of the distal tip.
Fig. 9 is a plan view showing an embodiment wherein outer circumferential surface of the distal tip is formed in an uneven shape.
Fig. 10 (A) and (B) are plan view and horizontal sectional view, respectively, showing further another embodiment of the distal tip.

### Description of Reference Numerals

- 1:: Ball head
- 2:: Stem
- 2a:: Distal end portion
- 2b:: Fitting hole
- 2c:: Fitting projection
- 2d:: Mounting hole
- 4:: Bone canal
- 5:: Porous surface
- 10:: Distal tip
- 11:: Fitting hole
- 12:: Male screw
- 13:: Female screw
- 14:: Fitting projection
- 15:: Minor fitting hole
- 16:: Ridge
- 17:: Bulge
- P:: Center axis
- Q:: Off-centered position
- L1:: Inner distance
- L2:: Outer distance
- L3:: Right-hand distance
- L4:: Left-hand distance

### DETAILED DESCRIPTION OF THE PREFERED EMBODIMENTS

In order to achieve the object described above, first feature of the present invention is a distal tip for the positioning of artificial joint stem, that is mounted near the distal end portion of stem of the cementless artificial joint thereby to guide the distal end portion of the stem so as not to make direct contact with the inner surface of the bone canal when the stem of the artificial joint is inserted into the bone canal, and make stable positioning of the stem distal end portion when the insertion of stem is completed, wherein the distal tip is made of a biodegradable and absorbable material.

Second feature of the distal tip for positioning of artificial joint stem of the present invention is, in addition to the first feature, that the distal tip is made in such a configuration as the diameter of the horizontal section thereof is larger than the diameter of the horizontal section of the stem near the distal end thereof, and the distal tip is provided with mounting means comprising fitting hole, fitting projection, screw or the like that engages with the stem.

Third feature of the distal tip for positioning of artificial joint stem of the present invention is, in addition to the second feature, that the mounting means for the distal tip is a fitting hole, a fitting protrusion or a screw provided on the distal tip at a position offset from the center axis of the distal tip.

Fourth feature of the distal tip for positioning of artificial joint stem of the present invention is, in addition to the second or third feature, that the mounting means for the distal tip is a fitting hole which has a projection or a ridge that protrudes from the inner circumferential surface toward the center of the fitting hole.

Fifth feature of the distal tip for positioning of artificial joint stem of the present invention is, in addition to any of the first through fourth features, that the distal tip is made in such a configuration as the outer circumference has polygonal or other uneven shape other than circle.

Sixth feature of the distal tip for positioning of artificial joint stem of the present invention is, in addition to the first feature, that the distal tip is made in pin shape, so as to be inserted in a plurality of mounting holes disposed at appropriate intervals along the circumference of the stem of the artificial joint near the distal end thereof.

Seventh feature of the distal tip for positioning of artificial joint stem of the present invention is, in addition to the sixth feature, that distal tips having different lengths are prepared, so that projecting lengths of the distal tips are different along the circumference when a plurality of distal tips are mounted at appropriate intervals along the circumference of the stem near the distal end thereof.

The distal tip for positioning of artificial joint stem according to the first feature enables it to guide the distal end portion of the stem so as not to make direct contact with the inner surface of the bone canal when the stem of the artificial joint is inserted into the bone canal, by mounting the distal tip at a position near the distal end portion of the stem of cementless artificial joint.

In addition, since the distal tip for positioning of artificial joint stem, which is provided for stable positioning of the stem distal end portion in the bone canal when the insertion of stem is completed, is made of a material that is decomposed and absorbed in living tissue, so that the distal tip can be decomposed and absorbed in a period of several months after the surgery during which the proximate portion of the stem and the cancellous bone develop rigid fixation with each other, it is made possible to eliminate the possibility of osteolysis in the cementless artificial joint and loosening of the artificial joint, pain and/or bone fracture due to the contact of the distal tip and the bone over a long period of time.

The distal tip for positioning of artificial joint stem according to the second feature enables it to guide the distal end portion of stem while reliably preventing direct contact between the distal end portion of the stem and the inner surface of the bone canal when the stem of the artificial joint is inserted into the bone canal, bymaking the diameter of the horizontal section of the distal tip larger than the diameter of the horizontal section of the stem near the distal end thereof.

Since the outer circumference of the distal tip having diameter larger than that of the stem near the distal end thereof is restricted by the inner surface of the bone canal from being displaced, position of the distal end portion of the stem is also regulated. Accordingly, stable positioning of the distal end portion of the stem in the bone canal is achieved by the distal chip when the insertion of the stem is completed.

The distal tip can be mounted easily and surely on the stem near the distal end thereof, by fitting the fitting hole, that is provided as the mounting means in the distal tip, on the stem from the outside, fitting the fitting protrusion, that is provided as the mounting means on the distal tip, into the fitting hole provided in the stem, or fitting the screw, that is provided as the mounting means on the distal tip, into the threaded hole provided in the stem, that is, by providing the mounting means on the distal tip in either case.

Moreover, since the distal tip is made of a biodegradable and bioabsorbable material, the distal tip can be decomposed and absorbed in a period of several months after the surgery during which the proximate portion of the stem and the cancellous bone develop rigid fixation with each other similarly to the case of the first feature, it is made possible to eliminate the possibility of osteolysis in the cementless artificial joint, loosening of the artificial joint, pain and/or bone fracture due to the contact of the distal tip and the bone over a long period of time.

According to the third feature, in addition to the effects of the second feature, it is made possible to make stable positioning of the distal end portion of the stem at a position which is offset from the center of the bone canal, by making the mounting means of the distal tip in the form of fitting hole, fitting projection or screw that is provided on the distal tip, and providing the mounting means at a position offset from the center axis of the distal tip. That is, since human bones are curved to degrees different from one individual to the other, there may be such a case that it is preferable to dispose the distal end portion of the stem in the bone canal at a position which is somewhat offset from the center rather at the center of the bone canal, in order to keep the stem at a favorable position where the inserted stem is held in well-balanced posture as a whole in the bone canal. Since such a condition of the bone can be known in advance through X-ray imaging, CT, MRI or the like, stable positioning of the distal end portion of the stem can be achieved at a predetermined position which is offset from the center of the bone canal by determining the preferable direction and amount of offsetting the distal end portion of the stem from the center of the bone canal beforehand, and accordingly fitting the distal tip into the stemwhile adjusting the azimuthal position of the distal tip in the horizontal plane.

According to the fourth feature, in addition to the effects of the second or third feature, it is made possible to fit the distal tip in the stem more firmly and securely than in a case in which the inner circumferential surface of the fitting hole is a smooth circular surface when fitting the distal tip into the stem, by making the mounting means of the distal tip in the form of fitting hole and providing a projection or a ridge that protrudes from the inner circumferential surface toward the center of the fitting hole. In addition, forming the projection or ridge in the fitting hole ensures the flow of bone marrow after the fitting hole is fixated with the stem.

According to the fifth feature, in addition to the effects of any of the first through the fourth feature, it is made possible to ensure sufficient flow of bone marrow in the bone canal without obstruction, since the outer circumference of the distal tip is formed in polygonal or other uneven shape other than circle.

According to the sixth feature, in addition to the effects of the first feature, it is made possible to easily mount the distal tip since the distal tip is formed in a pin shape that is inserted into the fitting hole of the artificial joint stem. Easy and stable positioning of the distal end portion of the stem at a predetermined position can be achieved with a portion of the stem near the distal end thereof being kept away from in the inner surface of the bone canal, by inserting the pin-shaped distal tips in a plurality of fitting holes that are provided at appropriate intervals in the circumferential direction of the stem near the distal end thereof.

According to the seventh feature, in addition to the effects of the sixth feature, it is made possible to achieve easy and stable positioning of the distal end of the stem at an off-centered position in the bone canal, by preparing pin-shaped distal tips having different lengths, mounting the distal tips at appropriate intervals along the circumference of the distal end portion of stem, so that the distal tips protrude from the stem by different lengths along the circumference.

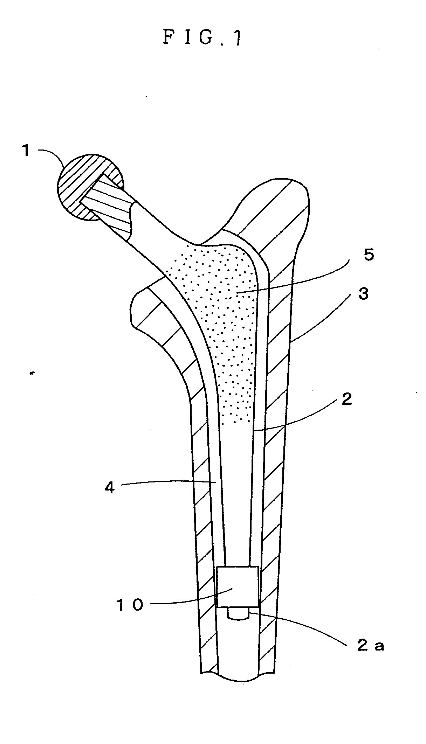

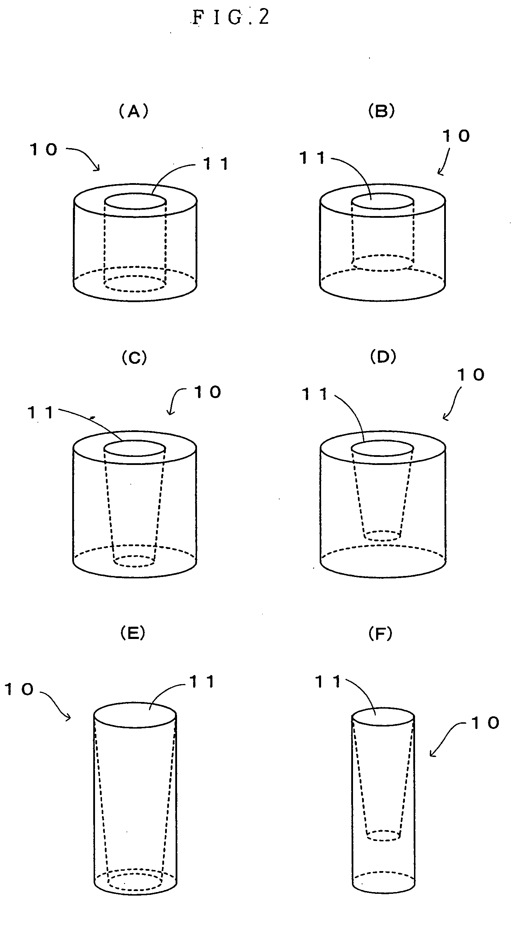

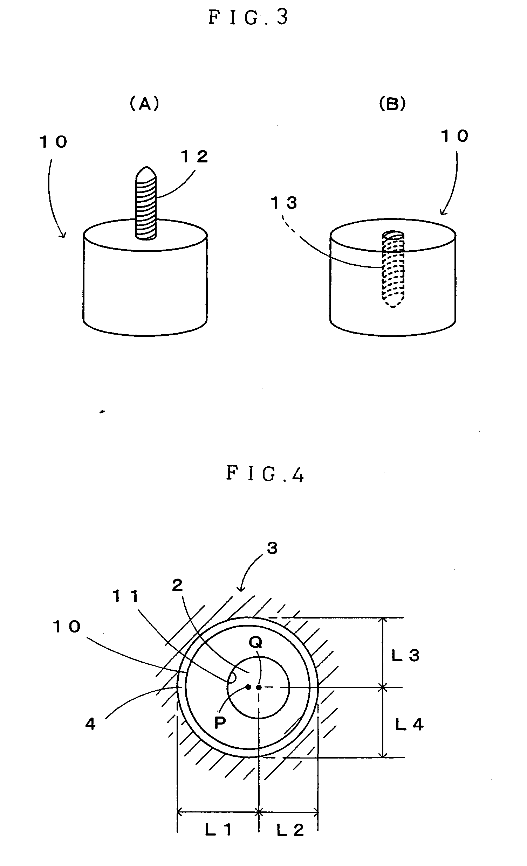

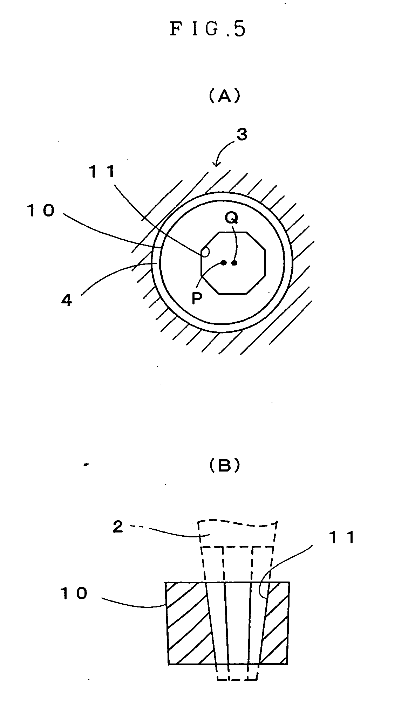

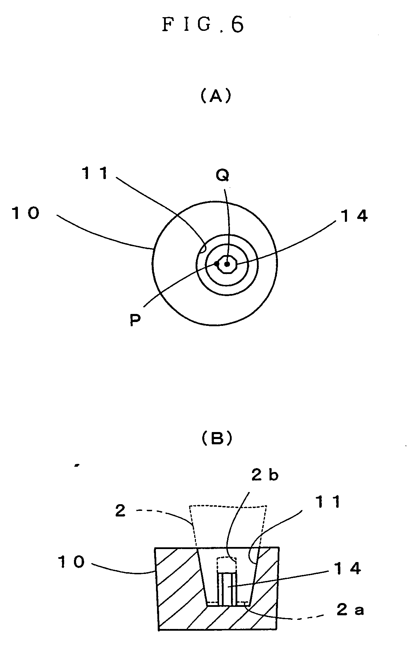

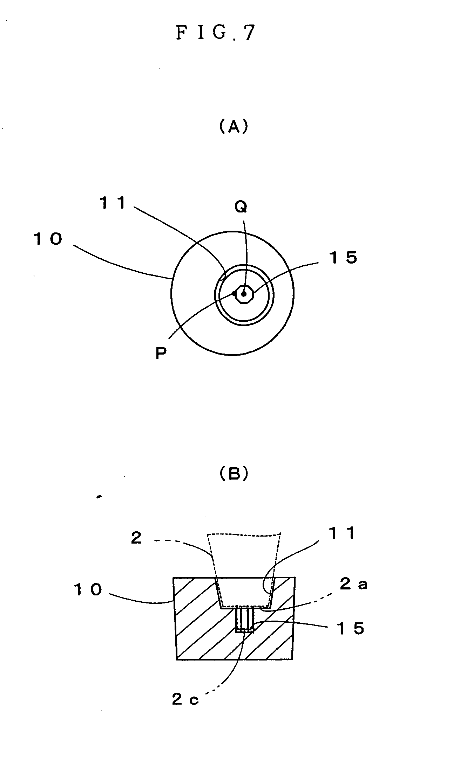

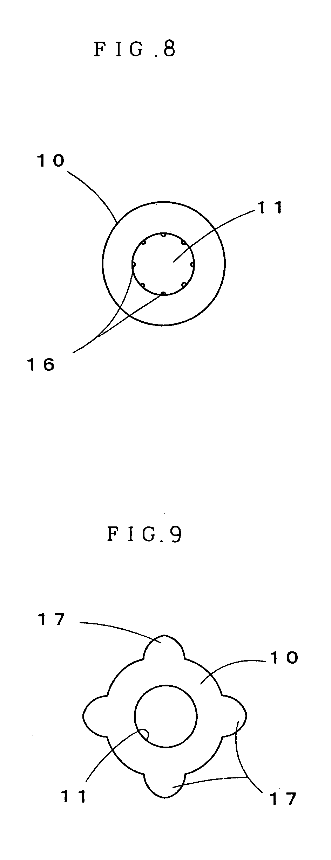

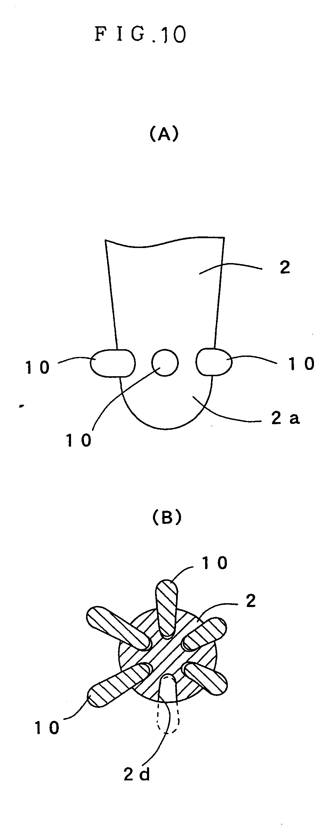

Fig. 1 is a sectional view showing the artificial hip joint stem having the distal tip of the present invention mounted thereon inserted in the bone canal of femoral bone. Fig. 2 (A) through (F) are perspective views showing various embodiments of the distal tip employing fitting hole. Fig. 3 (A) and (B) are perspective views showing embodiments of the distal tip of screw type. Fig. 4 is a plan view showing a distal tip of an embodiment wherein fitting hole of the distal tip is offset from the center axis thereof. Fig. 5 (A) and (B) are plan view and longitudinal sectional view, respectively, showing a distal tip of another embodiment wherein fitting hole of the distal tip is offset from the center axis thereof. Fig. 6 (A) and (B) are plan view and longitudinal sectional view, respectively, showing a distal tip of further another embodiment wherein fitting hole of the distal tip is offset from the center axis thereof. Fig. 7 (A) and (B) are plan view and longitudinal sectional view, respectively, showing a distal tip of further another embodiment wherein fitting hole of the distal tip is offset from the center axis thereof. Fig. 8 is a plan view showing an embodiment wherein a ridge protruding toward the center axis is formed on the inner circumference of the fitting hole of the distal tip. Fig. 9 is a plan view showing an embodiment wherein outer circumferential surface of the distal tip is formed in an uneven shape. Fig. 10 (A) and (B) are plan view and horizontal sectional view, respectively, showing further another embodiment of the distal tip.

First, with reference made to Fig. 1, reference numeral 1 denotes a ball head of artificial hip joint, 2 denotes a stem of the artificial hip joint, 3 denotes a proximal part of femoral bone, 4 denotes a bone canal and 5 denotes circumferential surface processed to be porous in the proximal portion of the artificial hip joint stem.

A distal tip 10 is mounted on the stem 2 of the artificial hip joint at a position near the distal end thereof. The artificial joint stem 2 having the distal tip 10 mounted thereon is inserted into the bone canal 4 from the proximal part of femoral bone 3 during surgery.

When the artificial joint stem 2 is inserted into the bone canal 4, the distal tip 10 guides the insertion of the stem 2 while preventing the distal end portion 2a of the stem 2 from making direct contact with the inner surface of the bone canal of the femoral bone 3 thereby causing a damage and from obstructing smooth insertion of the stem 2.

Diameter of horizontal section of the distal tip 10 is made larger than the diameter of horizontal section of the portion near the distal end of the stem 2.

When insertion of the artificial joint stem 2 into the bone canal 4 is completed, outer circumferential surface of the distal tip 10 faces the inner circumferential surface of the bone canal of the femoral bone 3, so that displacement of the distal tip 10 is constrained within a small region, thereby achieving stable positioning of the distal end portion 2a of the stem 2, too.

The stem 2 of the artificial joint may be made of a metallic material such as titanium alloy, cobalt -chromium alloy or stainless steel, but may also be made of other metallic material or non-metallic material having high strength.

The distal tip 10 is made of a biodegradable and absorbable material. For the material decomposed and absorbed in living tissue, PLLA (poly-L-lactic acid), PLA (poly-lactic acid),PGA (poly-glycolic acid),PDS (poly-dioxanone),
HA/PLLA (hydroxyapatite/PLLA) copolymer, PLLA/PGA copolymer or lactic acid-caprolactone copolymer may be used individually or two or more of these materials may be used in combination. It needs not to say that other biodegradable and absorbable material that is known or would be developed in the future may also be used.

The biodegradable and absorbable material is hydrolyzed and absorbed in the bone canal 4 over time. Duration in which the distal tip 10 performs its function can be controlled by adjusting the proportion of the material decomposed and absorbed in living tissue thereby controlling the rate of decomposition and absorption thereof in the living tissue. The duration in which the distal tip 10 performs its function refers to the period during which the cancellous bone grows onto the porous finished surface 5 of the proximate portion of the stem 2 and firmly joins therewith, so that the stem 2 and the femoral bone 3 develop rigid fixation with each other. This period is 3 to 6 months in average while it depends on the individual.

Now making reference to Fig. 2, configuration of the distal tip 10 is cylindrical as shown in Fig. 2(A), having a fitting hole 11 formed to penetrate therethrough along the center axis. In the case of the distal tip 10 shown in Fig. 2(A), the distal tip 10 is mounted at a position near the distal end portion of the stem 2 while passing through the distal end portion 2a of the stem 2, for example, as shown in Fig. 1. In this case, the stem 2 is properly tapered near the distal end portion 2a thereof, so as to fix the distal tip 10 thereby.

Configuration of the distal tip 10 may be cylindrical as shown in Fig. 2(B), having a fitting hole 11 that is formed along the center axis so as to stop midway without penetrating therethrough. The distal tip 10 of this configuration can be mounted on the stem 2 near the distal end thereof so as to cover the distal end portion 2a of the stem 2 from below.

Configuration of the distal tip 10 may be cylindrical as shown in Fig. 2(C), having a fitting hole 11 that is tapered off being formed to penetrate therethrough along the center axis. The distal tip 10 of this configuration can be satisfactorily mounted on the stem 2 which is not tapered near the distal end portion 2a thereof.

Configuration of the distal tip 10 may be cylindrical as shown in Fig. 2(D), having a fitting hole 11 that is tapered off being formed along the center axis so as to stop at a mid point thereof without penetrating. The distal tip 10 of this configuration can be mounted on the stem 2 so as to cover the distal end portion 2a of the stem 2 from below.

The distal tip 10 has such a configuration as shown in Fig. 2(E), (F) where the top end of the fitting hole 11 of the distal tip 10 has inner diameter near equal to the outer diameter of the top end of the fitting hole 11. This configuration makes it possible to connect the stem 2 and the distal tip 10 with substantially continuous surface without step at the joint.

Now referring to Fig. 3, the distal tip 10 may be mounted on the stem 2 near the distal end thereof also by means of screw. Fig. 3(A) shows a male screw 12 provided at the top end of the distal tip 10. With a female screw not shown in the drawing provided at the distal end portion 2a of the stem 2, the distal tip 10 is mounted by screwing therein. Fig. 3(B) shows a female screw 13 provided at the top end of the distal tip 10. With a male screw not shown in the drawing provided at the distal end portion 2a of stem 2, the distal tip 10 is mounted by screwing both members.

In the case of the distal tip 10 shown in Fig. 2 and Fig. 3, the fitting hole 11, the male screw 12 or the female screw 13 is provided along the center axis of the distal tip 10, although the fitting hole 11, the male screw 12 or the female screw 13 may also be provided at a position offset from the center axis of the distal tip 10.

Fig. 4 shows an embodiment in which the fitting hole 11 of the distal tip 10 is provided at a position Q offset from the center axis P of the distal tip 10. Offsetting the fitting hole 11 of the distal tip 10 from the center axis P of the distal tip 10 enables it to freely adjust the position of the stem 2, that is inserted into the fitting hole 11, in the bone canal 4.

In other words, in the distal end portion of stem 2 wherein the distal tip 10 is fitted, when the distance between the stem 2 and the inner circumferential surface of the femoral bone 3 is represented by inner distance L1, outer distance L2, right-hand distance L3 and left-hand distance L4, the distances L1, L2, L3 and L4 can be freely altered and adjusted. The alteration and adjustment can be made by turning the distal tip 10 by an angle within 360 degrees in the horizontal plane when fitting the distal tip 10 in the stem 2.

As well be seen from the description above, position of the distal end portion 2a of the stem 2 in the bone canal 4 can be freely adjusted so as to achieve positioning by using the distal tip 10 that has the off-centered fitting hole 11. This capability becomes very important when inserting the stem 2 into the bone canal 4 and positioning it with good balance as a whole in accordance with the curvature of the individual human bone. Moreover, the stem 2 can be disposed in the bone canal 4 with good balance as a whole in conjunction with the adjustment of the stem 2 position in the proximate portion (which can be done relatively easily because of visibility), when position of the distal end portion 2a of stem 2 in the bone canal 4 (position on horizontal plane in the bone canal 4) can be adjusted and disposed at a favorable position.

In the embodiment shown in Fig. 4, position of the mounting means in the form of fitting hole 11 is offset from the center axis P of the distal tip 10, although the fitting hole 11 may be replaced with a screw such as the male screw 12 or the female screw 13 of the distal tip 10 provided as the mounting means at a position offset from the center axis P.

The fitting hole 11 may also be replaced with a fitting protrusion provided as the mounting means at a position offset from the center axis P.

Fig. 5 shows the fitting hole 11 of the distal tip 10 provided at a position Q that is offset from the center axis P of the distal tip 10, similarly to the embodiment shown in Fig. 4, except for making the shape of the fitting hole 11, specifically the horizontal section thereof, in a polygonal form.

By employing the fitting hole 11 having polygonal shape, it is made possible to easily and surely fit and fix the distal tip with the distal end portion of the stem 2 having the same shape of horizontal section. Particularly, the extent of projection of the distal tip 10 around the stem 2 can be adjusted easily and reliably by turning the distal tip 10 in a step of the angle of one apex of the polygon.

Although the fitting hole 11 of the distal tip 10 is formed in the tapered shape in this embodiment, the fitting hole may not necessarily be tapered but may be a straight hole.

In the embodiment shown in Fig. 6, the off-centered fitting hole 11 of the distal tip 10 is formed with a bottom without penetrating therethrough, and a fitting projection 14 is provided so as to protrude from the bottom of the fitting hole 11, while the fitting projection 14 is inserted into the fitting hole 2b formed in the distal end portion 2a of stem 2 thereby to mount the distal tip 10 on the stem 2.

The fitting projection 14 is provided at a position Q offset from the center axis P of the distal tip 10. Position of the distal end portion 2a of the stem 2 can be adjusted in the bone canal 4 by fitting the fitting projection 14 into the fitting hole 2b while turning it in the circumferential direction and adjusting the position.

The fitting projection 14 is also made in a polygonal prism shape. Advantage of making it in a polygonal shape is the same as that described above.

In the embodiment shown in Fig. 7, the off-centered fitting hole 11 of the distal tip 10 is formed with a bottom without penetrating therethrough, and a minor fitting hole 15 is provided at the bottom of the fitting hole 11, so that a fitting projection 2c formed distal end portion 2a of stem is inserted into the minor fitting hole 15 thereby to mount the distal tip 10 on the stem 2.

The minor fitting hole 15 is provided at a position Q offset from the center axis P of the distal tip 10. Position of the distal end portion 2a of the stem 2 can be adjusted in the bone canal 4 by fitting the minor fitting hole 15 and the fitting projection 2c of the stem 2 while turning with respect to each other it in the circumferential direction and adjusting the position.

The minor fitting hole 15 is also made in a polygonal shape. Advantage of making it in a polygonal shape is the same as that described above.

As shown in Fig. 8, the fitting hole 11 of the distal tip 10 may be provided with a ridge 16 formed in the longitudinal direction to protrude from the inner circumferential surface thereof toward the center. A projection may also be provided instead of the ridge 16.

It is made possible to mount the distal tip 10 in the stem 2 more firmly and securely than in a case in which the inner circumferential surface of the fitting hole 11 is a smooth surface, by making the ridge 16 or projection that protrudes from the inner circumferential surface toward the center of the fitting hole 11 of the distal tip 10. By forming the inner circumference of the distal tip 10 in uneven shape, it is made possible to allow bone marrow or the like to flow freely through the clearance among the unevenness.

As shown in Fig. 9, the outer circumference of the distal tip 10 may not necessarily be circular. The outer circumference of the distal tip 10 may be formed in polygonal or other uneven shape In Fig. 9, bulges 17 are provided at equal intervals on the outer circumference of the distal tip 10. By forming the outer circumference of the distal tip 10 in uneven shape, it is made possible to allow bone marrow or the like to flow freely through the clearance among the unevenness.

Fi.10 shows another embodiment of the distal tip of the present invention. In this embodiment, the distal tip 10 is made in pin shape. A plurality of the pin-shaped distal tips 10 are prepared and are inserted into mounting holes 2d disposed at appropriate intervals (60 degrees in the case of this embodiment) along the circumference of the stem 2 near the distal end 2a thereof for mounting. With the plurality of pin-shaped distal tips 10 mounted in radial configuration on the circumference of the stem 2 near the distal end 2a thereof, the distal end portion 2a of the stem 2 can be positioned in stable condition while being separated from the inner surface of the bone canal 4.

The distal end of the pin-shaped distal tip 10 is formed in a round curved surface, so as to make soft contact with the inner surface of the bone canal 4.

Base-side end of the pin-shaped distal tip 10 may also be threaded so as to be mounted by screwing into the threaded mounting hole 2d of the stem 2.

The distal tips 10 may be provided in plurality having different lengths, so that projecting lengths of the pin-shaped distal tips 10 from the stem 2 are different along the circumference when the distal tips 10 of different lengths are mounted in the mounting holes 2d of the stem 2. This constitution enables it to make stable positioning of the distal end portion 2a of the stem 2 at an off-centered position in the bone canal 4. During the positioning, the off-centered position of the distal end portion 2a of the stem 2 in the bone canal 4 can be freely adjusted by selecting the position along the circumference of the stem 2 where one of the pin-shaped distal tips 10 of a particular length is to be mounted.

The distal tip 10 of the present invention may be applied, besides the artificial hip joint, to artificial shoulder joint, artificial elbow joint, artificial wrist joint, artificial finger joint, artificial knee joint and artificial ankle joint.

According to the present invention, having the constitution and operations described above, the distal tip for positioning of artificial joint stem described in claim 1 is mounted on the stem of cementless artificial joint at a position near the distal end thereof, so as to guide the artificial joint stem being inserted into the bone canal so that the distal end portion of the stem does not make direct contact with the inner surface of the bone canal, while achieving stable positioning of the stem distal end portion in the bone canal when insertion of the stem is completed, and the distal tip is made of a biodegradable and absorbable material, and therefore the artificial joint stem can be guided so that the stem distal end portion does not make direct contact with the inner surface of the bone canal when the stem of cementless artificial joint is inserted into the bone canal, and stable positioning of the stem distal end portion of the cementless artificial joint in the bone canal can be achieved.

Also because the distal tip for positioning of the artificial joint stem is made of a biodegradable and absorbable material, the distal tip can be decomposed and absorbed in a period of several months after the surgery during which the proximate portion of the stem and the cancellous bone develop rigid fixation, it is made possible to eliminate the possibility of osteolysis in the cementless artificial joint, loosening of the artificial joint, pain and/or bone fracture due to the contact of the distal tip and the bone over a long period of time.

According to the distal tip for positioning of the artificial joint stem described in claim 2, in addition to the effects of the constitution described in claim 1, because of such a constitution as the diameter of the horizontal section of the distal tip is made larger than the diameter of the horizontal section of the stem near the distal end thereof, and the mounting means comprising the fitting hole, the fitting projection, the screw or the like is provided, the artificial joint stem can be guided while reliably preventing the distal end portion of the stem from making direct contact with the inner surface of the bone canal when the artificial joint stem is inserted into the bone canal.

Moreover, since the stem is provided, at a position near the distal end thereof, with the distal tip having diameter larger than the diameter thereof, sufficiently stable positioning of the distal end portion of the stem can be achieved in the bone canal when insertion of the stem is completed.

Mounting on the stem can also be done easily and reliably by the mounting means comprising the fitting hole, the fitting projection, the screw or the like provided on the distal tip.

According to the distal tip for positioning of the artificial joint stem described in claim 3, in addition to the effects of the constitution described in claim 2, because of such a constitution as the mounting means for the distal tip is the fitting hole, the fitting projection, the screw or the like that is provided at a position which is offset from the center axis of the distal tip, the mounting means comprising the fitting hole, the fitting projection or the screw can be easily and reliably mounted at a position which is offset from the center axis of the distal tip.

Since the mounting means is installed at a position which is offset from the center axis of the distal tip, stable positioning of the distal end portion of the stem to be disposed in the bone canal can be made at a position which is offset from the center of the bone canal.

Since the position of the distal end portion of the stem can be offset from the center of the bone canal, the stem that has been inserted can be held favorably at a well-balanced position in accordance with the curvature of the individual human bone.

According to the distal tip for positioning of the artificial joint stem described in claim 4, in addition to the effects of the constitution described in claim 2 or 3, because the mounting means for the distal tip is provided in the form of fitting hole and the fitting hole is provided with the projection or ridge that protrudes from the inner circumferential surface toward the center of the fitting hole, the distal tip can be fitted into the stem more firmly and securely than in a case in which the inner circumferential surface of the fitting hole is a smooth surface. Moreover, the projection or ridge formed in the fitting hole ensures the flow of bone marrow even after the fitting hole has been fitted with the stem.

According to the distal tip for positioning of the artificial joint stem described in claim 5, in addition to the effects of the constitution described in one of claims 1 through 4, since the distal tip is made in such a configuration as the outer circumference has polygonal or other uneven shape other than circle, it is made possible to ensure sufficient flow of bone marrow in the bone canal without obstruction under the condition that the distal end portion of the stem is positioned in the bone canal by means of the distal tip.

According to the distal tip for positioning of the artificial joint stem described in claim 6, in addition to the effects of the constitution described in claim 1, since the distal tips are pin-shaped and are mounted by inserting into a plurality of fitting holes that are provided at appropriate intervals in the circumferential direction of the stem near the distal end of the artificial joint stem, easy and reliable positioning of the distal end portion of the stem in the bone canal can be achieved by using the pin-shaped distal tips. Furthermore, since the distal tips have pin shape, preparation for the implantation can be easily made by inserting the distal tip into the mounting hole of the stem.

According to the distal tip for positioning of the artificial joint stem described in claim 7, in addition to the effects of the constitution described in claim 6, since the distal tips having different lengths are prepared so that projecting lengths of the distal tips are different along the circumference when a plurality of distal tips are mounted at appropriate intervals along the circumference of the stem near the distal end thereof, free and reliable positioning of the distal end portion of the stem can be made very easily at an off-centered predetermined position in the bone canal by combining the pin-shaped distal tips of various lengths.

## Claims

1. A distal tip for the positioning of artificial joint stem that is mounted on a cementless artificial joint at a position near the distal end of the stem thereof and guides the distal end portion of the stem so as not to made direct contact with the inner surface of a bone canal when said artificial joint stem is inserted into the bone canal and, at the same time, achieves stable positioning of the distal end portion of the stem in the bone canal when the insertion of the step is completed, wherein said distal tip is made of a biodegradable and absorbable material.

2. The distal tip for the positioning of artificial joint stem according to claim 1, wherein the distal tip is made in such a configuration as the diameter of the horizontal section thereof is larger than the diameter of the horizontal section of the stem at a position near the distal end of the stem, and the distal tip has mounting means such as a fitting hole, fitting protrusion, screw or the like that fits with the stem.

3. The distal tip for the positioning of artificial joint stem according to claim 2, wherein the mounting means of the distal tip is a fitting hole, a fitting protrusion or a screw provided on the distal tip at a position that is offset from the center axis of the distal tip.

4. The distal tip for the positioning of artificial joint stem according to claim 2 or 3, wherein the mounting means of the distal tip is a fitting hole which has a projection or a ridge that protrudes from the inner circumferential surface toward the center of the fitting hole.

5. The distal tip for the positioning of artificial joint stem according to any one of claims 1 to 4, wherein the distal tip is made in such a configuration as the outer circumference has polygonal or other uneven shape other than circle.

6. The distal tip for the positioning of artificial joint stem according to claim 1, wherein the distal tip is made in pin shape, so as to be inserted in a plurality of mounting holes disposed at appropriate intervals along the circumference of the stem of the artificial joint near the distal end thereof.

7. The distal tip for the positioning of artificial joint stem according to claim 6, wherein the distal tips having different lengths are prepared, so that projecting length of the distal tip is different along the circumference when a plurality of distal tips are mounted at appropriate intervals along the circumference of the stem of the artificial joint near the distal end thereof.
